# EUROPÄISCHE PATENTANMELDUNG

(11) **EP 1 256 566 A2**
(43) Veröffentlichungstag der Anmeldung: **13.11.2002**
(21) Anmeldenummer: 02009184.9
(22) Anmeldetag: 25.04.2002
(51) Int. Cl.: C07C 67/08, C07C 69/704, C08K 5/11

(54) **Als Weichmacher verwendbare Zitronensäureester und ein Verfahren zu ihrer Herstellung**

(30) Priorität: 08.05.2001 DE 10122145
(71) Anmelder: Celanese Chemicals Europe GmbH, 60439 Frankfurt am Main (DE)
(72) Erfinder: Bohnen, Hans, Dr., 47441 Moers (DE); Klein, Thomas, 46149 Oberhausen (DE); Bergrath, Klaus, 46147 Oberhausen (DE)

(57) **Zusammenfassung**

Die vorliegende Erfindung betrifft Mischungen von Zitronensäureestern, die, bezogen auf die Gesamtmasse des Ester, 5 bis 40 Gew.-% Tri-n-butylcitrat, 59 bis 77 Gew.-% Zitronensäuremischester mit wenigstens einer n-Butylgruppe und 1 bis 18 Gew.-% eines Tri-C₄-C₁₀-Alkylcitrats, wobei n-Butyl ausgenommen ist, enthalten, sowie ihre Herstellung und ihre Verwendung als Weichmacher für thermoplastische Kunststoffe.

## Beschreibung

Die vorliegende Erfindung betrifft eine Mischung von Zitronensäureestern, ein Verfahren zur Ihrer Herstellung, sowie ihre Verwendung als Weichmacher.

Die erfindungsgemäßen Estermischungen zeichnen sich durch eine sehr geringe Flüchtigkeit aus und besitzen ein ausgezeichnetes Plastifiziervermögen für thermoplastische Kunststoffe und sind daher für Anwendungen im Kunststoffbereich hervorragend geeignet.

Weichmacher finden in großem Umfang Anwendungen in Kunststoffen, in Beschichtungsmitteln und Dichtungsmassen sowie in Kautschuk- und Gummiartikeln. Sie treten mit hochpolymeren thermoplastischen Stoffen, ohne chemisch zu reagieren, in physikalische Wechselwirkung, vorzugsweise durch ihr Löse- und Quellvermögen. Hierdurch bildet sich ein homogenes System aus, dessen thermoplastischer Bereich gegenüber den ursprünglichen Polymeren zu niedrigeren Temperaturen verschoben ist. Als Folge des Weichmacherzusatzes erhält man u.a. einen Werkstoff, dessen mechanische Eigenschaften gegenüber denen des unbehandelten Ausgangsmaterials optimiert sind. So werden z.B. Formänderungsvermögen, Elastizität und Festigkeit erhöht und die Härte verringert. Eine wichtige Weichmachereigenschaft besteht somit in dem Plastifiziervermögen von Kunststoffen. Um eine solche vorteilhafte Wirkung zu erzielen, müssen Weichmacher mit dem Kunststoff gut verträglich sein, um auch in hohen Mengen in die Kunststoffmasse eingearbeitet werden zu können.

Um Weichmachern eine möglichst weite Verwendung zu erschließen, müssen sie eine Reihe allgemein gültiger Kriterien erfüllen. Im Idealfall sollen sie geruchlos, farblos, licht- und wärmebeständig sein. Überdies verlangt man, dass sie unempfindlich gegenüber Wasser, schwer entflammbar, schwer brennbar und wenig flüchtig sind und nur eine geringe Migrationstendenz aus der Kunststoffmasse bei der bestimmungsgemäßen Verwendung aufweisen. Besonders der geringen Flüchtigkeit der Weichmacher, die sowohl bei der Einarbeitung in die Kunststoffmasse als auch bei der Verwendung der Formkörper in der Praxis zu beachten ist, kommt eine besondere Bedeutung zu. Darüber hinaus müssen für Anwendungen im Lebensmittelbereich und im medizinischen Gebiet vorgesehene Weichmacher gesundheitlich unbedenklich sein. Schließlich soll die Herstellung der Weichmacher sowohl hinsichtlich der benötigten apparativen Ausrüstung als auch hinsichtlich der erforderlichen Verfahrensschritte einfach sein und, um ökologischen Anforderungen zu genügen, die Bildung nichtverwertbarer Nebenprodukte und schädlicher Abfallstoffe vermeiden.

Aufgrund ihrer ausgezeichneten Weichmachereigenschaften finden bestimmte Phthalate umfangreiche Anwendungen als Zusatzstoffe für Thermoplaste, insbesondere für PVC. Ihrem universellen Einsatz steht jedoch entgegen, dass diese wiederholt gesundheitliche Bedenken begegnen. So verbietet sich ihr Einsatz in Verbindung mit Lebensmitteln, z.B. als Verpackungsmaterial und in anderen Erzeugnissen, deren Gebrauch aus Gründen der Gesundheitsvorsorge besonderer Sorgfalt unterworfen ist. Zu ihnen gehören Artikel des täglichen Bedarfs, wie Haushaltsgegenstände und Gegenstände für die Versorgung und Betreuung von Kindern, darin eingeschlossen Spielzeug, sowie Produkte, die im medizinischen Bereich eingesetzt werden. Man verwendet daher als Weichmacher für Hilfs- und Fertigprodukte aus Thermoplasten, die für diese speziellen Anwendungsgebiete bestimmt sind, nicht Phthalate sondern die toxisch unbedenklichen Zitronensäureester.

Es ist bekannt, Zitronensäureester auf Basis eines einheitlichen Alkohols, beispielsweise auf Basis von n-Butanol oder 2-Ethylhexanol einzusetzen (z.B. Gächter/Müller, Kunststoffadditive, 3.Ausgabe 1990, Carl Hanser-Verlag, Seiten 417-418). Häufig erfahren diese Ester noch eine weitere Derivatisierung, indem die freie Hydroxylgruppe der Zitronensäure durch Reaktion mit einer Carbonsäure oder einem Carbonsäurederivat, beispielsweise mit dem Anhydrid, acyliert wird. Ein solches Acylierungsverfahren ist beispielsweise aus der DE-AS-1 099 523 bekannt. Aus der DE-A1-35 20 750 sind aber auch solche Zitronensäureester, bzw. ihre acylierten Formen bekannt, in denen unterschiedliche Alkohole an der Zitronensäure gebunden sind, wie beispielsweise das Acetyl-tri-n-(hexyl/octyl/decyl)-citrat. Die bekannten Mischester werden für die Herstellung von Medizinartikeln verwendet.

Zitronensäureester dürfen nur eine geringe Flüchtigkeit zeigen, damit es bei der Verarbeitung nur zu möglichst geringen Produktverlusten und zu einer möglichst geringen Belastung der Arbeitsluft durch Abgase kommt. Sie müssen aber auf der anderen Seite eine gute Verträglichkeit mit dem Kunststoff aufweisen, damit sie auch in hohen Mengen in den thermoplastischen Kunststoff eingearbeitet werden können und es selbst bei längerem Gebrauch zu keinem Ausschwitzen der Weichmacher aus der thermoplastischen Kunststoffmasse kommt. Die bisher bekannten Zitronensäureester erfüllen gleichzeitig diese beiden Anforderungen noch nicht zur vollsten Zufriedenheit.

Es bestand daher die Aufgabe Zitronensäureester bereitzustellen, die sowohl eine geringe Flüchtigkeit besitzen und gleichzeitig eine hervorragende Verträglichkeit mit einem thermoplastischen Kunststoff zeigen, so daß sie in großen Mengen in den thermoplastischen Kunststoff eingearbeitet werden können.

Diese Aufgabe wird gelöst durch eine Mischung von Zitronensäureestern enthaltend, bezogen auf die Gesamtmasse des Esters, 5 bis 40 Gew.-% Tri-n-butylcitrat, 59 bis 77 Gew.-% der Verbindung der allgemeinen Formel (I) 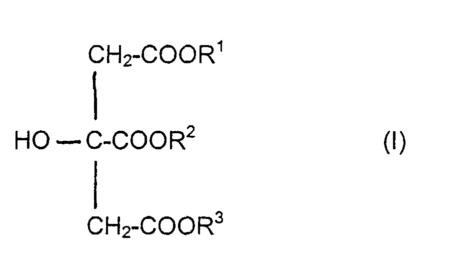 in der R¹, R² und R³ einen geradkettigen oder verzweigten Alkylrest mit 4 bis 10 Kohlenstoffatomen bedeuten, mit der Maßgabe, dass wenigstens einer der Reste R¹, R² oder R³ gleich n-Butyl ist und die beiden anderen Reste gleich oder verschieden sind, aber nicht alle Reste R¹, R² und R³ gleichzeitig n-Butyl sind, und 1 bis 18 Gew.-% der Verbindung der allgemeinen Formel (I), mit der Maßgabe, dass R¹, R² und R³ gleich oder verschieden aber nicht n-Butyl sind.

Überraschenderweise zeigen die erfindungsgemäßen Zitronensäureestermischungen sowohl ein ausgezeichnetes niedriges Flüchtigkeitsverhalten als auch eine hervorragende Verträglichkeit mit dem thermoplastischen Kunststoff. Sie können daher in großen Mengen in Kunststoffe eingearbeitet werden und eignen sich daher besonders für die Herstellung solcher Formkörper, die sehr flexibel sein müssen, beispielsweise für flexible Schläuche und Behälter, die in der Medizintechnik Verwendung finden.

Die neben n-Butanol verwendete Alkoholkomponente ist iso-Butanol oder ein aliphatischer Monoalkohol mit 5 bis 10 Kohlenstoffatomen aus einer beliebigen Provenienz. Wegen ihrer leichten Verfügbarkeit finden insbesondere Oxoalkohole Anwendung, d.h. Alkohole, die durch Oxosynthese, also durch Umsetzung von Monoolefinen mit Kohlenmonoxid und Wasserstoff hergestellt wurden. Vorzugsweise verwendet man n-Pentanol, n-Heptanol, 2-Ethylhexanol, n-Oktanol, n-Decanol oder iso-Decanole, beispielsweise 2-Propylheptanol.

Vorzugsweise liegt die Zusammensetzung der erfindungsgemäßen Estermischung, bezogen auf die Gesamtmasse des Esters, bei 8 bis 30 Gew.-% an Tri-n-butylcitrat, 67 bis 75 Gew.-% der Verbindung der allgemeinen Formel (I), mit der Maßgabe, dass wenigstens einer der Reste R¹, R² oder R³ gleich n-Butyl ist und die beiden anderen Reste gleich oder verschieden sind aber nicht alle Reste R¹, R² und R³ gleichzeitig n-Butyl sind, und 3 bis 17 Gew.-% der Verbindung der allgemeinen Formel (I) mit der Maßgabe, dass R¹, R² und R³ gleich oder verschieden aber nicht n-Butyl sind.

Als besonders geeignet hat sich eine Estermischung erwiesen, in der die zweite Alkoholkomponente 2-Ethylhexanol ist und die, bezogen auf die Gesamtmasse des Esters, 26 Gew.-% Tri-n-butylcitrat, 45 Gew.-% Di-n-butyl-2-ethylhexylcitrat, 25 Gew.-% n-Butyl-di-2-ethylhexylcitrat und 4 Gew.-% Tri-2-ethylhexylcitrat enthält.

Die Messung der Flüchtigkeit erfolgt mit Hilfe eines Brabender Schnellwasserbestimmers mit rotierendem Teller. Dabei wird an einer Flüssigkeitsprobe der Gewichtsverlust bestimmt, den die Probe bei einer thermischen Behandlung bei 150°C über einen Zeitraum von 2 Stunden erfährt.

Das Plastifiziervermögen wird mittels der kritischen Lösetemperatur gemäß DIN 53408 bestimmt, wobei zur Messung die PVC-Type S-1067 verwendet wird.

Überraschenderweise zeigen die erfindungsgemäßen Estermischungen eine deutlich niedrigere Flüchtigkeit, als man nach der routinemäßigen Interpolation zwischen den an Tri-n-butylcitrat und an der Verbindung der Formel (I) gemessenen Werten erwarten würde, in der R¹, R² und R³ gleich oder verschieden aber nicht gleich n-Butyl sind. Bei der kritischen Lösetemperatur beobachtet man einen nahezu linearen Verlauf mit zunehmendem Temperaturanstieg in Richtung der Verbindung, in der R¹, R² und R³ gleich oder verschieden aber nicht gleich n-Butyl sind. Die erfindungsgemäßen Estermischungen lösen daher die Aufgabe, Zitronensäureester bereitzustellen, die ein verbessertes Flüchtigkeitsverhalten bei noch sehr gutem Plastifiziervermögen aufweisen.

Die Herstellung der erfindungsgemäßen Estermischung erfolgt durch Reaktion der Zitronensäure mit dem n-Butanol enthaltenden Alkoholgemisch. Die Umsetzung wird mit überschüssigem Alkoholgemisch in Gegenwart von Katalysatoren durchgeführt, um eine möglichst vollständige Umsetzung der Säure in einer vertretbaren Zeit zu erzielen. Im allgemeinen ist das Molverhältnis an Zitronensäure zur Alkoholmischung größer als 1:3,6, wobei sich dieses Molverhältnis auf die in der Alkoholmischung vorliegenden Hydroxylgruppen bezieht. Für einen hohen Umsatz, und damit verbunden für eine hohe Produktausbeute, ist es zweckmäßig, neben der Verwendung eines Alkoholüberschusses auch das gebildete Reaktionswasser laufend zu entfernen. Als Katalysatoren haben sich Säuren, die im Reaktionsgemisch gelöst oder suspendiert vorliegen können, bewährt. Der Estersynthese schließt sich die Entfernung des Katalysators und die Wäsche des Produktes mit Wasser an. Daraufhin wird der überschüssige Alkohol aus dem Reaktionsgemisch mittels Destillation abgetrennt. Anschließend wird der Rückstand bei vermindertem Druck und erhöhter Temperatur getrocknet, gegebenenfalls mit Hilfe eines Inertgasstromes. Die erfindungsgemäßen Mischester fallen als wasserklare Flüssigkeiten im Rückstand des Trocknungsprozesses an.

Zur Herstellung der erfindungsgemäßen Estermischungen verestert man eine Alkoholmischung, die einen n-Butanolgehalt von 50 bis 90 Mol-% und einen Gehalt an der zweiten Monoalkoholkomponente R'-OH von 10 bis 50 Mol-% aufweist. Die zweite Monoalkoholkomponente kann entweder eine einheitliche Verbindung sein, wobei R' gleich R¹ gleich R² oder gleich R³ ist und R¹, R² und R³ nicht gleichzeitig n-Butyl bedeuten. Die zweite Alkoholkomponente kann auch eine Mischung aus zwei verschiedenen Monoalkoholen, ausgenommen n-Butanol, sein. Besonders bewährt hat es sich, eine Alkoholmischung einzusetzen, die 50 bis 70 Mol-% n-Butanol und 30 bis 50 Mol-% des einheitlichen Monoalkohols R'-OH enthält. Insbesondere verwendet man zur Veresterung eine Mischung aus n-Butanol und 2-Ethylhexanol oder aus n-Butanol und iso-Decanol, beispielsweise 2-Propylheptanol.

Als Temperatur, bei der die Umsetzung der Zitronensäure und der Alkoholmischung vorgenommen wird, hat sich der Bereich von 110 bis 140°C bewährt. Niedrigere Temperaturen sind nicht ausgeschlossen, sofern aufgrund der Eigenart der Reaktionspartner oder der Reaktionsumstände eine genügend hohe Reaktionsgeschwindigkeit erzielt wird oder nur Teilumsätze angestrebt werden. Höhere Temperaturen vermeidet man im allgemeinen, um das Risiko der Zersetzung von Ausgangsstoffen, Neben- und Endprodukten und damit der Verunreinigung des Mischesters, z.B, durch farbschädigende Stoffe oder durch Aconitsäure, die durch Wasserabspaltung aus der Zitronensäure gebildet wird, vorzubeugen. Die Anwendung von vermindertem Druck während der Reaktion ist möglich, solche Ausgestaltung des Verfahrens wird sich jedoch auf Sonderfälle beschränken.

Um unter wirtschaftlichen Aspekten vertretbare Reaktionszeiten sicherzustellen, ist es erforderlich, die Geschwindigkeit der Umsetzung von Säure und Alkohol durch Zusatz eines Katalysators zu erhöhen. Geeignet sind für diesen Zweck die üblichen, katalytisch wirksamen Substanzen wie Titanate, Schwefelsäure, Ameisensäure, Polyphosphorsäure, Methansulfonsäure oder para-Toluolsulfonsäure, die im Reaktionsgemisch gelöst oder suspendiert als reine Verbindung oder auch in Form eines Gemisches verschiedener Substanzen eingesetzt werden. Bevorzugt werden Schwefelsäure, Methansulfonsäure oder para-Toluolsulfonsäure, die preiswert zur Verfügung stehen und aus dem Reaktionsgemisch leicht entfernt werden können. Die Menge des eingesetzten Katalysators kann sich über einen weiten Bereich erstrecken. Es können z.B. sowohl 0,01 Gew.-% als auch 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,01 bis 1,0, vorzugsweise 0,01 bis 0,5 Gew.-%, jeweils bezogen auf das Reaktionsgemisch.

Die Entfernung des Reaktionswassers aus dem Reaktionsgemisch, die erforderlich ist, um das Veresterungsgleichgewicht in Richtung des gewünschten Produktes zu verschieben, erfolgt mit Hilfe von Azeotropbildnern (Schleppmittel). Üblicherweise wählt man hierfür organische Lösungsmittel aus, die mit Wasser entsprechende, im Bereich der Reaktionstemperatur von 110 bis 140°C siedende, Mischungen bilden. Beispiele für geeignete Schleppmittel sind Hexan, Cyclohexan, Toluol und die isomeren Xylole, bevorzugt wird Cyclohexan. Die zur vollständigen Abtrennung des Wassers erforderliche Menge Schleppmittel läßt sich aus entsprechend der Stöchiometrie der Veresterungsreaktion berechneten Wasserbildung und aus der Zusammensetzung des binären Azeotrops in einfacher Weise ermitteln. Es hat sich bewährt, das Schleppmittel im Überschuß einzusetzen, zweckmäßig in einem Anteil, der 50 bis 200 Gew.-% über der theoretisch berechneten Menge liegt. Durch eine gezielte Auswahl der Schleppmittelmenge innerhalb dieses Bereiches, kann die Veresterungstemperatur innerhalb des Intervalls von 110° bis 140°C auf den gewünschten Wert eingestellt werden. Durch Auffangen und Abtrennen des abdestillierten Schleppmittel-/Wassergemisches läßt sich in einfacher Weise der Fortgang der Reaktion verfolgen. Das aus dem Azeotrop abgeschiedene Schleppmittel kann unmittelbar in die Reaktion zurückgeführt werden.

Im Anschluß an die Veresterungsreaktion wird der saure Katalysator durch Zugabe alkalischer Reagenzien, zum Beispiel durch Zugabe einer wäßrigen Natriumhydroxid-, Natriumcarbonat- oder Natriumhydrogencarbonatlösung neutralisiert, in dem man den Rohester innig mit der alkalischen Lösung vermischt. Der nach Phasentrennung erhaltene Rohester wird zur Entfernung letzter Alkalispuren mit Wasser gewaschen. Anschließend destilliert man den Alkoholüberschuß sowie Reste des Schleppmittels ab und trocknet den Destillationsrückstand bei vermindertem Druck und erhöhter Temperatur, gegebenenfalls durch Einleiten eines Inertgases.

Die durch Umsetzung der Zitronensäure und dem Alkoholgemisch gewonnenen Triester enthalten noch eine freie Hydroxylgruppe. Es ist bekannt, daß sich die Verträglichkeit der Zitronensäuretriester in einem thermoplastischen Kunststoff erhöhen läßt, wenn die Hydroxylgruppe in eine Acylgruppe derivatisiert wird. Weiterhin erhöht sich die thermische Stabilität der Zitronensäureester durch das Einführen der Acylgruppe, da bei thermischer Belastung die freie Hydroxylgruppe unter Wasserbildung aus den Zitronensäureestern eliminiert werden kann und zu ungesättigten Verbindungen führt, die als Verunreinigungen im Esterprodukt unerwünscht sind.

Ein weiterer Gegenstand der Erfindung betrifft demnach eine Mischung acylierter Zitronensäureester, in der die in der erfindungsgemäßen Estermischung vorliegenden Zitronensäureester in acylierter Form vorliegen, wobei die Acylgruppe linear oder verzweigt sein kann und 2 bis 5 Kohlenstoffatome enthält.

Zur Acylierung der in der erfindungsgemäßen Estermischung enthaltenen hydroxylgruppenhaltigen Ester verwendet man eine lineare oder verzweigte Carbonsäure mit 2 bis 5 Kohlenstoffatomen im Molekül oder ihr Derivat, vorzugsweise das entsprechende Anhydrid oder das Säurechlorid. Besonders bewährt hat sich Essigsäureanhydrid, Propionsäureanhydrid oder Buttersäureanhydrid als Acylierungsmittel.

Sind die acylierten Verbindungen das Zielprodukt, so setzt ihre Herstellung zweckmäßigerweise bei dem nach Neutralisation, Wasserwäsche und Abdestillation des Alkoholüberschusses und etwaiger Schleppmittelreste erhaltenen Rohesters an.

Die Veresterung der freien Hydroxylgruppe erfolgt mit der Carbonsäure oder dem Carbonsäurederivat, vorzugsweise mit dem Säureanhydrid, die, bezogen auf den Triester, im Überschuß eingesetzt werden. Es ist vorteilhaft, je Mol Triester 1,2 bis 1,8 Mol, vorzugsweise 1,3 bis 1,5 Mol einer Monocarbonsäure, bzw. die entsprechende Menge des Säurederivats anzuwenden. Zweckmäßig hält man die Reaktionstemperatur bis 110°C ein, wobei die jeweils auszuwählende aktuelle Temperatur sich nach der Reaktivität des Esters und des Acylierungsmittels richtet. Bevorzugt wird der Temperaturbereich von 60 bis 80°C. Im allgemeinen setzt man dem Reaktionsgemisch wiederum einen Katalysator zu, wobei man in der Acylierungsstufe solche Katalysatoren einsetzt, die sich auch in der Veresterungsstufe bewährt haben. Die Menge an Katalysator beträgt dabei im allgemeinen 0,01 bis 5 Gew.-%, vorzugsweise 0,01 bis 1,0 und besonders bevorzugt 0,01 bis 0,5 Gew.-%, jeweils bezogen auf das Reaktionsgemisch.

Nach erfolgter Acylierung werden aus dem Rohgemisch zunächst überschüssiges Acylierungsmittel und weitere flüchtige Verbindungen, beispielsweise die aus dem Acylierungsmittel freigesetzte Carbonsäure, falls man mit einem Anhydrid als Acylierungsmittel arbeitet, abdestilliert. Der Destillation schließt sich die Neutralisation des rohen Acylierungsgemischs an, die analog der im Anschluß an die Veresterungsreaktion erfolgten Neutralisation durchgeführt wird. Nach Wasserwäsche wird das Produkt zur Entfernung letzter Feuchtigkeitsspuren nach konventionellen Verfahren getrocknet. So kann man z.B. die Wasserreste bei mäßig erhöhten Temperaturen unter vermindertem Druck entfernen oder den Strom eines inerten Gases, wie Stickstoff durch den Rückstand leiten.

In den nachfolgenden Beispielen wird das erfindungsgemäße Verfahren näher erläutert, die neue Arbeitsweise ist jedoch nicht auf diese eine Ausführungsform beschränkt.

### Beispiele

### Beispiel 1

### Herstellung von 70/30 Citrat

In einem Dreihalskolben, der mit Rührer, Innenthermometer und Wasserabscheider ausgestattet ist, wurden 1681,0 g Citronensäuremonohydrat (8 mol), 1491,8 g n-Butanol (20,2 mol), 1118,0 g 2-Ethylhexanol (8,6 mol) sowie 9,0 g Methansulfonsäure (0,1 mol) und 252 g Cyclohexan vorgelegt und solange gerührt, bis eine homogene Lösung entstand. Das Reaktionsgemisch wurde anschließend auf 120°C erhitzt und das anfallende Reaktionswasser über einen Zeitraum von 9 Stunden bei dieser Temperatur aus dem Reaktionsgemisch entfernt. Nach Reaktionsende wurde der Ansatz durch Zugabe von 201,8 g Wasser und 162,9 g einer 3 Gew.-%-igen wäßrigen NaOH-Lösung neutralisiert. Nach Phasentrennung wurde die organische Produktphase mit Wasser nachgewaschen. Zur Abtrennung des überschüssigen Alkoholanteils wurde der Rohester einer Wasserdampfdestillation unterzogen und anschließend zur Abtrennung von Wasserresten bei 120°C und einem Druck von etwa 100 Pa im Stickstoffstrom getrocknet. Die erhaltene Estermischung wies folgende, gaschromatographisch ermittelte Zusammensetzung auf.

| GC-Zusammensetzung der Estermischung (Gew.-%) | |
|---|---|
| Tri-n-butylcitrat | 25,6 |
| Di-n-butyl-2-ethylhexylcitrat | 44,8 |
| n-Butyl-di-2-ethylhexylcitrat | 24,5 |
| Tri-2-ethylhexylcitrat | 4,3 |
| Rest | 0,8 |

### Beispiel 2

### Herstellung von 50/50 Citrat

In einem Dreihalskolben, der mit Rührer, Innenthermometer und Wasserabscheider ausgestattet ist, wurden 2101,4 g Citronensäuremonohydrat (10 mol), 1334,2 g n-Butanol (18 mol), 2340,0 g 2-Ethylhexanol (18 mol) sowie 10,0 g Methansulfonsäure (0,1 mol) und 200 g Cyclohexan vorgelegt und solange gerührt, bis eine homogene Lösung entstand. Das Reaktionsgemisch wurde anschließend auf 120°C erhitzt und das anfallende Reaktionswasser über einen Zeitraum von 15 Stunden bei dieser Temperatur aus dem Reaktionsgemisch entfernt. Nach Reaktionsende wurde der Ansatz durch Zugabe von 160 g Wasser und 55,2 g einer 20 Gew.-%-igen wäßrigen NaOH-Lösung neutralisiert und die organische Produktphase nach der Phasentrennung mit Wasser nachgewaschen. Zur Abtrennung des überschüssigen Alkoholanteils wurde der Rohester einer Wasserdampfdestillation unterzogen und anschließend zur Abtrennung der Wasserreste bei 120°C und einem Druck von etwa 100 Pa im Stickstoffstrom getrocknet. Die erhaltene Estermischung wies folgende gaschromatographisch ermittelte Zusammensetzung auf.

| GC-Zusammensetzung der Estermischung (Gew.-%) | |
|---|---|
| Tri-n-butylcitrat | 8,1 |
| Di-n-butyl-2-ethylhexylcitrat | 32,8 |
| n-Butyl-di-2-ethylhexylcitrat | 42,0 |
| Tri-2-ethylhexylcitrat | 16,0 |
| Rest | 0,2 |

An den erhaltenen Estermischungen wurden die Flüchtigkeitswerte im Brabender Schnellwasserbestimmer (150°C/2 Stunden) und die kritische Lösetemperatur gemäß DIN 53408 mit der PVC-Type S 1067 bestimmt. Für Vergleichszwecke wurde auch reines Tri-n-butylcitrat und Tri-2-ethylhexylcitrat vermessen. Die Meßergebnisse sind aus der nachfolgenden Tabelle ersichtlich.

**Tabelle 1:**

| Flüchtigkeit und kritische Lösetemperatur von Zitronensäureestern | | | |
|---|---|---|---|
| Nummer | Zitronensäureester | Flüchtigkeit (%) | kritische Lösetemperatur (°C) |
| 1 | Tri-n-butylcitrat | 2,48 | 108 |
| 2 | 70/30 Citrat (Beispiel 1) | 0,95 | 127 |
| 3 | 50/50 Citrat (Beispiel 2) | 0,69 | 138 |
| 4 | Tri-2-ethylhexylcitrat | 0,23 | 177 |

Wie aus der Tabelle 1 ersichtlich, liegen die an den erfindungsgemäßen Estermischungen (Beispiel 2 und 3) gemessenen Flüchtigkeitswerte deutlich unter den Flüchtigkeitswerten, die man aus einer routinemäßigen Interpolation zwischen den für Tri-n-butylcitrat und Tri-2-ethylhexylcitrat gemessenen Flüchtigkeitswerten erwarten würde. Ausgehend von reinem Tri-n-butylcitrat, das auf der einen Seite zwar ein gutes Plastifiziervermögen aufweist aber auf der anderen Seite eine hohe Flüchtigkeit besitzt und bei der Brabender Schnellwasserbestimmung einen Gewichtsverlust von 2,5 Gew.-% erleidet, läßt sich der Flüchtigkeitswert auf etwa 1,0 Gew.-% erniedrigen, wenn man von einer Estermischung ausgeht, die ca. 26 Gew.-% Tri-n-butylcitrat, 4 Gew.-% Tri-2-ethylhexylcitrat und 70 Gew.-% Mischester enthält. Die kritische Lösetemperatur steigt dabei lediglich von 108°C auf 127°C an. Während die gemessene kritische Lösetemperatur sich im Rahmen der interpolierten Steigerung zwischen den Eckwerten des reinen Tri-n-butylcitrats und Tri-2-ethylhexylcitrats bewegt, ist der Flüchtigkeitsrückgang deutlich stärker ausgeprägt als man nach der interpolierten Flüchtigkeitskurve erwarten würde. Ein derartiger beobachteter Flüchtigkeitsrückgang konnte anhand der interpolierten Flüchtigkeitswerte nicht erwartet werden.

## Patentansprüche

1. Mischung von Zitronensäureestern enthaltend, bezogen auf die Gesamtmasse des Esters, 5 bis 40 Gew.-% Tri-n-butylcitrat, 59 bis 77 Gew.-% der Verbindung der allgemeinen Formel (I) 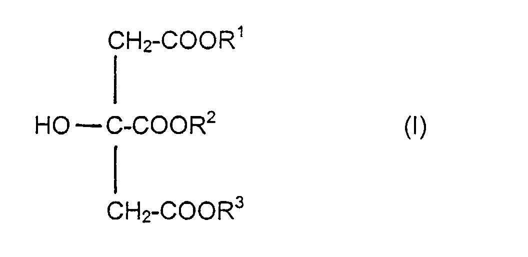 in der R¹, R² und R³ einen geradkettigen oder verzweigten Alkylrest mit 4 bis 10 Kohlenstoffatomen bedeuten, mit der Maßgabe, dass wenigstens einer der Reste R¹, R² oder R³ gleich n-Butyl ist und die beiden anderen Reste gleich oder verschieden sind, aber nicht alle Reste R¹, R² und R³ gleichzeitig n-Butyl sind, und 1 bis 18 Gew.-% der Verbindung der allgemeinen Formel (I), mit der Maßgabe, dass R¹, R² und R³ gleich oder verschieden aber nicht n-Butyl sind.

2. Mischungen gemäß Anspruch 1, **dadurch gekennzeichnet, dass** sie, bezogen auf die Gesamtmasse des Esters, 8 bis 30 Gew.-% an Tri-n-butylcitrat, 67 bis 75 Gew.-% der Verbindung der allgemeinen Formel (I), mit der Maßgabe, dass wenigstens einer der Reste R¹, R² oder R³ gleich n-Butyl ist und die beiden anderen Reste gleich oder verschieden sind aber nicht alle Reste R¹, R² und R³ gleichzeitig n-Butyl sind, und 3 bis 17 Gew.-% der Verbindung der allgemeinen Formel (I) mit der Maßgabe, dass R¹, R² und R³ gleich oder verschieden aber nicht n-Butyl sind, enthalten.

3. Mischungen nach Anspruch 1 oder 2, **dadurch gekennzeichnet, dass** R¹, R² und R³ gleich oder verschieden ist und n-Pentyl, n-Heptyl, 2-Ethylhexyl, n-Oktyl, n-Decyl, iso-Decyl, vorzugsweise 2-Propylheptyl, bedeutet.

4. Mischung von Zitronensäureestern enthaltend, bezogen auf die Gesamtmasse des Esters, 26 Gew.-% Tri-n-butylcitrat, 45 Gew.-% Di-n-butyl-2-ethylhexylcitrat, 25 Gew.-% n-Butyl-di-2-ethylhexylcitrat und 4 Gew.-% Tri-2-ethylhexylcitrat.

5. Mischungen acylierter Zitronensäureester, **dadurch gekennzeichnet, dass** die Zitronensäureester in der Mischung gemäß Anspruch 1 eine lineare oder verzweigte Acylgruppe mit 2 bis 5 Kohlenstoffatomen enthalten.

6. Mischungen gemäß Anspruch 5, **dadurch gekennzeichnet, daß** sich die Acylgruppe vom Acetyl-, Propionyl- oder Butyryl-Rest ableitet.

7. Verfahren zur Herstellung von Mischungen der Zitronensäureester gemäß einem der Ansprüche 1 bis 4, umfassend
(a) die Reaktion der Zitronensäure mit einem Butanol enthaltenden Alkoholgemisch in Gegenwart von Katalysatoren unter Entfernung des gebildeten Reaktionswassers mit Hilfe eines Schleppmittels; und
(b) Entfernung des Katalysators, Wäsche des Produktes mit Wasser, Abtrennung des überschüssigen Alkohols und Trocknung bei vermindertem Druck und erhöhter Temperatur.

8. Verfahren nach Anspruch 7, **dadurch gekennzeichnet, daß** man eine Mischung aus n-Butanol und 2-Ethylhexanol mit Zitronensäure verestert.

9. Verfahren nach Anspruch 7, **dadurch gekennzeichnet, daß** man eine Mischung aus n-Butanol und iso-Decanol mit Zitronensäure verestert.

10. Verfahren zur Herstellung von Mischungen acylierter Zitronensäureester gemäß Anspruch 5, umfassend
(a) die Reaktion der Zitronensäure mit einem Butanol enthaltenden Alkoholgemisch in Gegenwart von Katalysatoren unter Entfernung des gebildeten Reaktionswassers mit Hilfe eines Schleppmittels;
(b) Entfernung des Katalysators, Wäsche des Produktes mit Wasser, Abtrennung des überschüssigen Alkohols;
(c) Acylierung der freien Hydroxylgruppe der Zitronensäureester in Gegenwart eines Katalysators mit einer linearen oder verzweigten Monocarbonsäure mit 2 bis 5 Kohlenstoffatomen im Molekül oder einem Derivat dieser Monocarbonsäure;
(d) Entfernung des überschüssigen Acylierungsmittels und seiner Umsetzungsprodukte;
(e) Neutralisation des in der Acylierungsstufe verwendeten Katalysators, Wasserwäsche und Trocknung bei vermindertem Druck und erhöhter Temperatur.

11. Verfahren nach Anspruch 10, **dadurch gekennzeichnet, dass** man in der Acylierungsstufe (c) Essigsäureanhydrid, Propionsäureanhydrid oder Buttersäureanhydrid verwendet.

12. Verfahren nach Anspruch 10 oder 11, **dadurch gekennzeichnet, dass** man eine Mischung aus n-Butanol und 2-Ethylhexanol mit Zitronensäure verestert.

13. Verfahren nach Anspruch 10 oder 11, **dadurch gekennzeichnet, dass** man eine Mischung aus n-Butanol und iso-Decanol mit Zitronensäure verestert.

14. Verwendung der Mischungen von Zitronensäureestern gemäß einem der Ansprüche 1 bis 4 als Weichmacher für thermoplastische Kunststoffe.

15. Verwendung der Mischungen von acylierten Zitronensäureestem gemäß den Ansprüche 5 oder 6 als Weichmacher für thermoplastische Kunststoffe.

16. Plastifizierungsmittel enthaltend eine Estermischung der Zusammensetzung gemäß einem der Ansprüche 1 bis 4.

17. Plastifizierungsmittel enthaltend eine Estermischung der Zusammensetzung gemäß Anspruch 5 oder 6.
